# EUROPÄISCHE PATENTSCHRIFT

(11) **EP 1 362 511 B1**
(45) Veröffentlichungstag und Bekanntmachung des Hinweises auf die Patenterteilung: **30.03.2011**
(21) Anmeldenummer: 03010783.3
(22) Anmeldetag: 14.05.2003
(51) Int. Cl.: A01N 1/02

(54) **Protektive Lösung zur Verhinderung von Ischämieschäden**
Protective solution for preventing ischaemia
Solution protectrice pour la prévention de l'ischémie

(30) Priorität: 17.05.2002 DE 10222561
(43) Veröffentlichungstag der Anmeldung: 19.11.2003
(60) Teilanmeldung: 07012466.4
(73) Patentinhaber: Dr. Franz Köhler Chemie GmbH, 64665 Alsbach-Hähnlein (DE)
(72) Erfinder: Bruns, Wilfried, 68647 Biblis (DE); Köhler, Gernot, 64665 Alsbach-Hähnlein (DE); De Groot, Herbert, 40591 Düsseldorf (DE); Rauen, Ursula, 45327 Essen (DE)
(74) Vertreter: Blumbach - Zinngrebe

(56) Entgegenhaltungen:
- EP-A- 0 054 635
- WO-A-88/09168
- WO-A-92/08453
- WO-A-99/36055

## Beschreibung

Die vorliegende Erfindung betrifft eine verbesserte Zusammensetzung von Lösungen zur Organprotektion, vorzugsweise von Herz, Lunge, Niere, Leber, Pankreas und Gefäßsystemen, um gegebenenfalls langandauernde Operationen am nicht-durchbluteten ischämischen Organ durchrühren zu können, zur Konservierung derselben Organe zur Transplantation mit reduzierter Schädigung des Gewebes gegenüber anderen Konservierungsverfahren während der Transport- bzw. Lagerzeit oder zur Reperfusion ischämischer Organe. Die vorliegende Erfindung stellt hierfür geeignete Lösungen sowie Verfahren zu deren Herstellung nach den Anforderungen der GMP-Richtlinien bereit.

Mit der Einführung des hyperkalämischen Herzstillstandes durch Melrose im Jahre 1955 waren die Herzchirurgen in der Lage, komplexe Eingriffe am blutleeren, nichtschlagenden Herzen durchzuführen. Wenngleich mit den damaligen kardioplegischen Lösungen nur bis zu 40 Minuten operiert werden konnte, waren erstmalig operative Rekonstruktionen bei angeborenen Herzmissbildungen und der Einsatz von Herzklappen-Prothesen möglich. Einen Meilenstein der Herzchirurgie setzte Banard 1967 mit der erstmals durchgeführten Herztransplantation.

Ziel der weiteren Forschung bestand darin, die bis dahin limitierte Operationsdauer mit geeigneten Lösungen und entsprechenden Anwendungsverfahren zu verlängern. Schon in den 60iger Jahren konnte die Arbeitsgruppe von Bretschneider mit neuen Konzepten zur Verbesserung der Myocard-Protektion die Ischämiezeiten erheblich verlängern. Erstmalig wird durch Entzug von Natrium der Herzstillstand induziert und mit Substraten, wie z. B. Procain. Acetylcholin und Novocain die Zellmembran geschützt, um der Entstehung eines intrazellulären Ödems entgegen zu wirken.

In der europäischen Patentschrift 12272 ist eine protektive Lösung für Herz. Niere und andere Organe beschrieben, welche durch ein Puffersystem auf der Basis von Histidin + Histidin-HCl gekennzeichnet ist und welche außerdem Natrium-, Kalium- und Magnesium-Ionen sowie ein Polyol oder einen Zucker enthält. Mit dieser protektiven Lösung wird die tolerierbare Ischämiezeit um den Faktor 8 gegenüber den Zeiten von unbehandelten Herzen erzielt. Eine weitere Verbesserung dieser Lösung wird in der Europäischen Patentschrift 54635 beschrieben, wonach durch den Zusatz von α-Ketoglutarat der aerobe Stoffwechsel während der ca. 8 bis 10 Minuten andauernden Perfusion des Organs mit der protektiven Lösung durch die günstige Beeinflussung des Citratzyklus der ATP-Verlust verringert wird. In den darauffolgenden Jahren richtete sich das Augenmerk der Kliniker und Physiologen auf die zeitliche Phase der Beendigung der Ischämie, die mit der Erwärmung des hypothermen und hypoxischen Organs und der Reperfusion mit Blut beendet ist, und in der die Organe ihre verständige Funktion wiedererlangen. Studien hierzu zeigen, dass gerade in der sog. Reperfusionsphase verschiedene pathophysiologische Prozesse ablaufen, die mit dem Begriff Reperfusionsschaden (I-R-Schziden) zusammengefasst werden. Dabei kommt es vor allem zu Endothelzellschäden, die zum Teil als Ursache, zum Teil als Folge entzündlicher Prozesse interpretiert werden und in deren Pathogenese reaktiven Saucrstoffspezies eine erhebliche Bedeutung zuzukommen scheint. In den letztes Jahren stellte sich zudem die zum Schutz der Organe eingesetzte Kälte als eigenständiger Schädigungsfaktor heraus; keine der derzeit eingesetzten Konservierungslösungen vermag gegen diese Schädigung einen Schutz zu bieten.

Zweck der vorliegenden Erfindung ist es, die folgenden pathuphysiologischen Vorgänge während der Ischämie und der Reperfusion zu verhindern bzw. zu vermindern:
- ischänlische Schädigung
- Kälteschädigung (kälteinduzierte Apoptose)
- Reperfusionsschäden
- Entzündliche Prozesse.

Dazu ist in dem Patentanspruch 1 eine organprotektive Lösung angegeben, die der Erfüllung der genannten Aufgabe dient. Aus den dargestellten Mechanismen der Zell- und Gewebeschädigung bei Kardioplegie und Organkonservierung ergeben sich eine Reihe von Forderungen für die Zusammensetzung einer Organprotektionslösung. Für die Realisierung dieser Forderungen haben wir Substanzen gefunden, die die Konzeption der neuartigen, effektiv wirksamen Organprotektionslösungen gemäß Patentanspruch 1 erlauben. Die Zusammensetzung der Lösung ist solcherart gewählt, dass sie vor allen zuvor genannten Schädigungskomponenten einen wirksamen Schutz bietet. Durch die mechanismenorientierten und nicht toxischen Komponenten kann die Konservierungsschädigung deutlich vermindert werden. Die von den bisher verwendeten Konservierungslösungen bekannte Toxizität bei (akzidenteller bzw. während der Anastomosierungszeit erfolgender) Erwärmung des Organs wird vermieden. Auf diese Weise werden die Funktionalität und Viabilität des entsprechenden Organgewebes verbessert und die Ischämie- und Kaltlagerungstoleranz erhöht; damit werden längere Organkonservierungszeiten möglich. Dies kann auch einen Beitrag zur Logistik leisten, um die Verfügbarkeit von Organen für die Transplantation zu verbessern.

Es erweist sich als vorteilhaft, wenn ein Hydroxamsäurederivat eingesetzt wird, bei dem das Wasserstoffatom am Hydroxamsäurestickstoff alkyl-, aryl- oder alkylarylsubstituiert mit C₁ bis C₂₀ ist.

Ferner empfiehlt es sich, im Hydroxamsäurederivat das Wasserstoffatom am Hydroxamsäurekohlenstoff alkyl-, aryl- oder alkylaryl substituiert ist mit C₁ bis C₂₀, wobei dieser Substituent auch Heteroatome und/oder Hydroxy-, Amino-, Methoxy-Gruppen enthalten kann.

Günstig ist es auch, wenn die Substituenten am Hydroxamsäurestickstoff mit den Sustituenten am Hydroxamkohlenstoff zu einem Ring geschlossen sind.

Für die Erfindung empfiehlt es sich, eine oder mehrere der Verbindungen und/oder deren Salze aus der Gruppe
Acethydroxamsäure,
Acet-N-methylhydroxamsäure,
N-Benzylacethydroxamsäure,
Hexanhydroxamsäure,
Hexan-N-methylhydroxamsäure,
Benzhydroxamsäure,
N-Methylbenzhydroxamsäure;
Salicylhydroxamsäure,
Salicyl-N-methylhydroxamsäure,
Salicyl-N-benzylhydroxamsäure,
2-Phenylacethydroxamsäure
2-Phenylacet-N-methylhydroxamsäure
3,4-Dimethoxybenzhydroxamsäure
3,4-Dimethoxy-N-methyl-benzhydroxamsäure
2,3-Dimethoxy-N-methyl-benzhydroxamsäure
2,4-Dimethoxy-N-methyl-benzhydroxamsäure
3,5-Dimethoxy-N-methyl-benzhydroxamsäure
2,4-Dihydroxybenzhydroxamsäure
2,3-Dihydroxybenzhydroxamsäure
3,4-Dihydroxybenzhydroxamsäure
3,4,5-Trimethoxy-benzhydroxamsäure
3,4,5-Trimethoxy-N-methyl-benzhydroxamsäure
4-Hydroxy-3-methoxy-benzhydroxamsäure
2-Hydroxy-3-methoxy-benzhydroxamsäure
2-Hydroxy-5-methoxy-benzhydroxamsäure
2-Hydroxy-3-methyl-isocarbostyril
4-Chlor-N-methyl-benzhydroxamsäure
6-Cyclohexy-1-hydroxy-4-methyl-2( 1H)-pyridon
enthält.

Ferner kommt ein Zusatz an Deferoxamin mit Vorteil in Betracht, wobei sich eine Konzentration an Deferoxamin von bis zu etwa 10 mmol/l empfiehlt.

Empfehlenswert ist ein Zusatz an einem Derivat der 6-Hydroxy-2,5,7,8-tetramethylchroman-2-carbonsäure oder dem entsprechenden Methylester.

Zweckmäßig ist der Puffer auf der Basis von N-Acetylhistidin, insbesondere auf der Basis N-acylierten Histidins, gegebenenfalls in Kombination mit einer geeigneten organischen Base, z.B. N-Glycylhistidin/ N-Glycylhistidin-hydrochlorid oder N-Acetylhistidin/Lysin und/oder Arginin und/oder Cholin.

Vorteilhafterweise enthält die Lösung einen Kationengehalt an Lysin und/oder Lysinderivat, bevorzugt lysinhaltige Dipeptide, sowie gegebenenfalls einen Anionengehalt an Aspartat.

Zweckmäßig ist die Hydroxamsäure und/oder ihre Derivate in einer Konzentration von bis zu etwa 10 mmol/l in der Lösung enthalten.

Günstig ist auch, wenn das Trolox in einer Konzentration von bis zu etwa 10 mmol/l in der Lösung enthalten ist.

Vorteilhaft ist das N-Acetylhistidin/N-Acethylhistidin- in einer Konzentration von etwa 20 mmol/l bis etwa 265 mmol/l enthalten.

Es empfiehlt sich ferner in der Lösung als Elektrolyt Natrium zweckmäßig in einer Konzentration von etwa 10 mmol/l bis etwa 120 mmol/l vorzusehen. Alternativ oder ergänzend kann Kalium zweckmäßig in einer Konzentration von etwa 5 mmol/l bis etwa 25 mmol/l enthalten sein.

Ferner sollte Magnesium zweckmäßig in einer Konzentration von etwa 3 mmol/l bis etwa 27 mmol/l enthalten sein.

Schließlich ist Calcium in einer freien Konzentration von zweckmäßig etwa 0,0001 mmol/l bis etwa 1,5 mmol/l mit Vorteil enthalten.

Besonders zu empfehlen ist Lysin und/oder dessen Derivate, vorzugsweise als ein lysinhaltiges Dipeptid, zweckmäßig in einer Konzentration bis zu etwa 140 mmol/l.

Ein Gehalt von Aspartat in einer Konzentration von bis zu etwa 140 mmol/l in der Lösung ist vorteilhaft.

Sofern in der Lösung Chlorid enthalten ist, empfiehlt es sich, das Aspartat im Verhältnis zum Chlorid im Überschuss vorzusehen.

Bewährt hat sich auch ein Gehalt an α-Ketoglutarat zweckmäßig in einer Konzentration von etwa 1 mmol/l bis etwa 9 mmol/l.

Vorteilhafterweise ist Osmolyt zweckmäßig in einer Konzentration von bis zu etwa 140 mmol/l in der Lösung enthalten

Zur Herstellung einer Lösung nach einem der vorstehenden Ansprüche empfiehlt sich ein Verfahren, bei welchem in einem Überschuß an Wasser, zweckmäßig in etwa 90% der benötigten Wassermenge, die Elektrolyten unter Rühren aufgelöst, der Puffer und anschließend und die Hydroxamsäure und/oder deren Derivate zugegeben werden, sodann der pH-Wert eingestellt wird, der oder die Osmolyte zugegeben und die Lösung auf das Sollvolumen mit Wässer aufgefüllt wird.

Zur Gewinung der Hydroxamsäure und/oder deren Derivate können zweckmäßig niedere Alkohole, und/oder DMF und/oder THF als Lösungsmittel eingesetzt werden.

Das Verfahren gestaltet sich besonders günstig, wenn die Umsetzung mit einem Carbonsäureester basenkatalysiert durchgeführt wird.

Die Erfindung kann zur Linderung oder Vermeidung von Reperfusionsschäden nach Herzinfarkt, Schlaganfall, unfallchirurgischen Eingriffen oder F,xtrernitätenreperfusion eingesetzt werden. Ferner empfiehlt sich ihre Verwendung in der Therapie von Erkrankungen, die ihre Ursache in einer Hyperferrämie (z.B. Alzheimer), in einer Zellschädigung durch Radikale, Sauerstoffradikale oder H₂O₂ haben.

Eisenchelatoren können zur Hemmung der eisenabhängigen Kälteschädigung bzw. der kälteinduzierten Apoptose dienen. Als Verbindungen zur Verminderung dieser Zellschädigungen ist nicht jeder Komplexbildner geeignet. So ist z. B. EDTA wie auch das im Custodiol enthaltene Histidin hier weniger unwirksam, obwohl auch diese Liganden starke Eisenkomplexe bilden. Das Eisen muß vielmehr in spezieller Weise vom Liganden gebunden werden, und der Ligand muß die intrazellulären Kompartimente schnell und in ausreichender Konzentration erreichen.

Es konnte gezeigt werden, daß dem Strukturelement der Hydroxamsäure I bzw. 11, welches im Deferoxamin dreimal enthalten ist, als geeignetem Strukturelement bzw. Ligand für Eisen eine besondere Bedeutung zukommt, 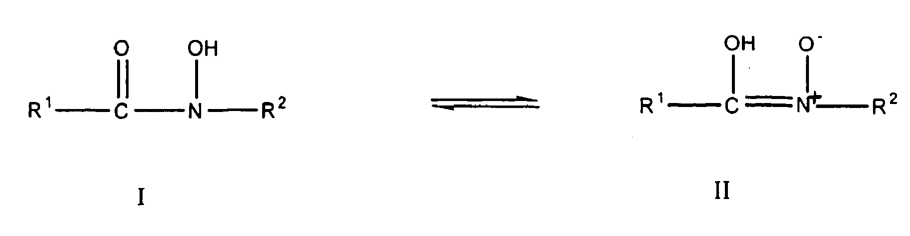 wobei R¹ = C₁ bis C₂₀ Alkyl , Aryl, Alkyl-aryl geradkettig oder verzweigt,bedeutet auch Heteroatome und/oder weitere Substituenten wie -OH, -NH₂ etc. umfassen kann und R² = H bedeutet und sonst wie R¹ sein kann und wobei R¹ und R² zu einem Ring geschlossen sein und/oder weitere Substituenten enthalten können, wie z.B. 2-Hydroxypyridin-N-oxid und Derivate, die lediglich eine andere tautomere Struktur des Falles darstellen, wobei R¹ und R² zu einem Ring mit konjugierten Doppelbindungen geschlossen sein können. Mit den im Vergleich zum Deferoxamin kleinen und lipophilen Substanzen ist die Strategie einer schnellen intrazellulären Verfügbarkeit eines starken Eisenchelators realisiert.

Auch einfache Hydroxamsäuren zeigen schon positive Wirkung; jedoch ist für eine gute Wirksamkeit ein bestimmtes Hydrophilie/Lipophilie-Verhältnis erforderlich. Darüberhinaus konnte gezeigt werden, daß die am Hydroxamsäurestickstoff alkylsubstituierten (R² = Alkyl) gegenüber den unsubstituierten Verbindungen (R² = H) generell wirksamer sind. Ist R² eine stark elektronenziehende Gruppe wie z.B. - CO - R³ (R³ gleiche Bedeutung wie R¹) führt dies zur Unwirksamkeit, obwohl auch diese Verbindungen noch leicht Eisenkomplexe bilden, wie z.B.:
N-Hydroxysuccinimid
3,4-Dihydro-3-hydroxy-4-oxo-1,2,3-benzotriazin

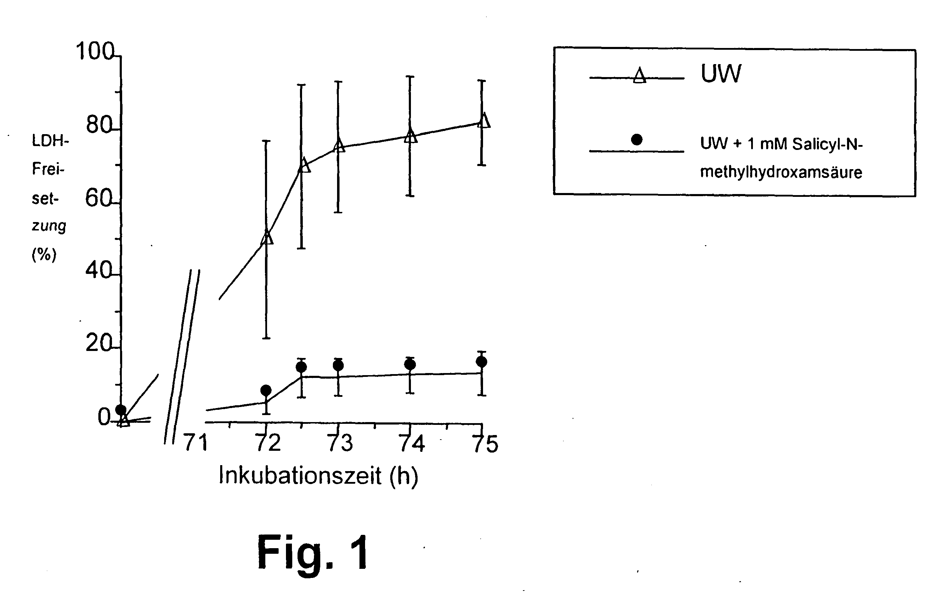

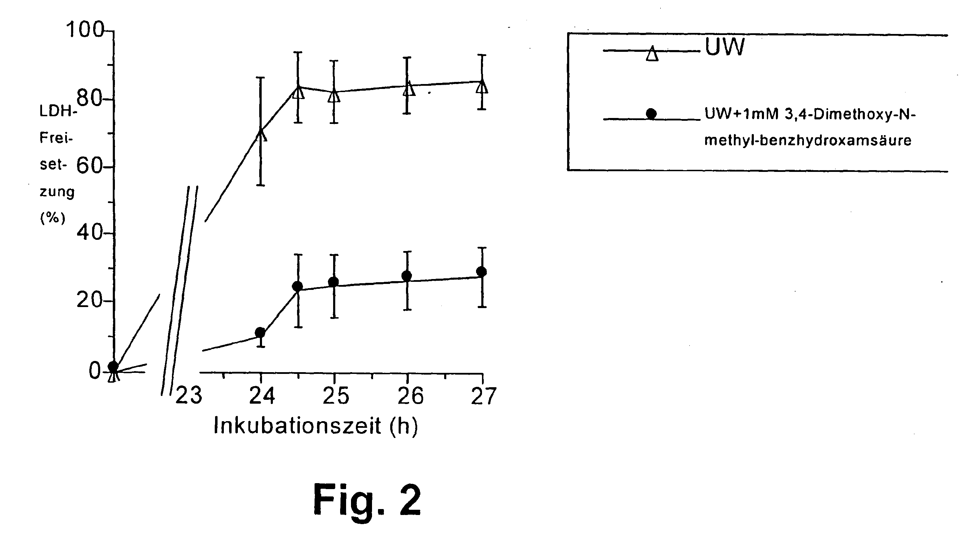

Die Wirksamkeit zweier Beispielsubstanzen demonstrieren die Abbildungen 1 und 2.

Die Hydroxamsäure und/oder Derivate können daher in den erfindungsgemäßen Lösungen enthalten sein, zweckmäßig in Konzentrationen bis zu etwa 10 mmol/l.

Fig. 1 zeigt die Hemmung der Kälteschädigung von Leberendothelzellen durch Salicyl-N-methylhydroxamsäure. Kultivierte Rattenleberendothelzellen wurden 72 h bei 4°C in University of Wisconsin-Lösung (UW) unter aeroben Bedingungen in Gegenwart und Abwesenheit von I mM Salicyl-N-methylhydroxamsäure inkubiert und anschließend für 3 h im Zellkulturmedium wiedererwärmt (37°C). Als Parameter der Zellschädigung diente die Freisetzung cytosolischer Lactatdehydrogenase (LDH).

Fig. 2 zeigt die Hemmung der Kälteschädigung von Hepatozyten durch 3,4-Dimethoxy-N-methyl-benzhydroxamsäure.Kultivierte Rattenhepatozyten wurden 24 h bei 4°C in University of Wisconsin-Lösung (UW) unter aeroben Bedingungen in Gegenwart und Abwesenheit von 1 mM 3,4-Dimethoxy-N-methyl-benzhydroxamsäure inkubiert und anschließend für 3 h im Zellkulturmedium wiedererwärmt (37°C). Als Parameter der Zellschädigung diente die Freisetzung cytosolischer Lactatdehydrogenase (LDH).

Deferoxamin ist ein starker Eisenchelator, der als sechszähniger Ligand das Eisen in nicht redoxaktiver Form bindet. Es dient damit einem optimalen Schutz bei längerer Kälteexposition. Deferoxamin ist jedoch ein relativ großes, hydrophiles Molekül mit einer dadurch begrenzten Membrangängigkeit. Deferoxamin erreicht deshalb nicht alle intrazellulären Kompartimente in ausreichender Konzentration und Geschwindigkeit, um einen kompletten Schutz zu bieten. Es kann in einer Konzentration von vorzugsweise bis zu etwa 10 mmol/l in der Lösung enthalten sein.

Weitere Verbindungen, die zweckmäßig der erfindungsgemäßen organprotektiven Lösung zugesetzt werden können, sind in Patentanspruch 3 angegeben.

In einer weiteren Ausführungsform der Erfindung wird der anspruchsgemäßen Lösung 6-Hydroxy-2,5,7,8-tetramethylchrom-2-carbonsäure (Trolox) oder ein Derivat zugesetzt. Der relativ hydrophile, aber membrangängige Radikalfänger Trolox bzw. seine Derivate davon werden zugesetzt, um die bei der Kälteschädigung und bei der Reoxygenierungschädigung entstehenden intrazellulären Radikale abzufangen. Das Troloxderivat liegt in der erfindungsgemäßen Lösung zweckmäßig in einer Konzentration von bis zu etwa 10 mmol/l vor.

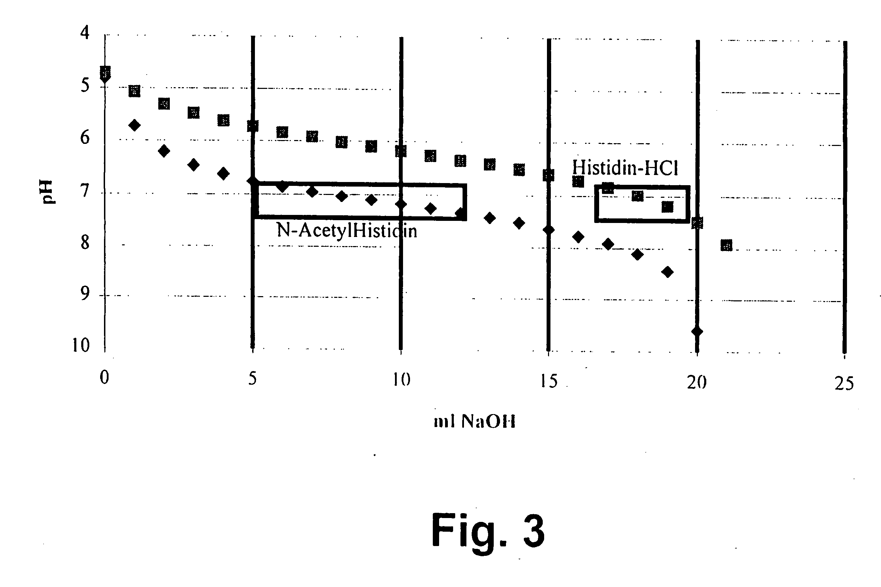

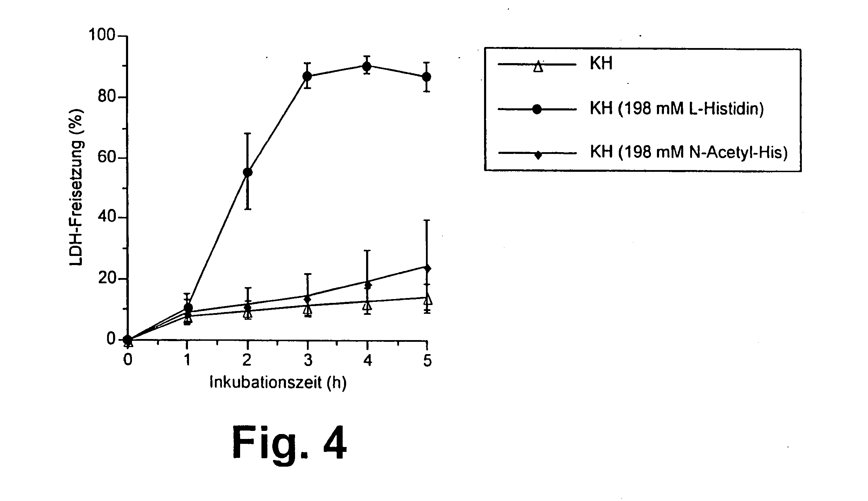

Alle organprotektiven Lösungen verfügen über Puffersubstanzen, die der unvermeidlichen Azidose während der Ischämie entgegenwirken. Phosphate oder Bicarbonate sind hierbei die meistverwendeten anorganischen Puffersysteme. Als absolut überlegen bezüglich der Pufferkapazität und pH-Stabilität hat sich das in Custodiol® eingesetzte organische Puffersystem Histidin/Histidin-HCl erwiesen. Besondere Vorteile ergeben sich, wenn Derivate des Histidins, wie z.B. N-Acetylhistidin, statt Histidin/Histidin HCl als Puffersystem in der vorstehend genannten aber auch in anderen bekannten protcktiven Lösungen benutzt werden. Figur 3 zeigt eine um den Faktor 2 verbesserte Pufferkapazität des N-Acetylhistidins im physiologischen Bereich im Vergleich zu Histidin/Histidin HCl. Durch den Einsatz des Histidin Derivats weden auch einige unerwünschte Nebenwirkungen des Histidins (Toxizität insbesondere in der Wärme aber auch in der Kälte bei einigen Zelltypen wie z.B. bei Hepatozyten) vermieden ohne die gute Pufferkapazität zu beeinträchtigen (Figur 4).

Fig. 3 zeigt die jeweilige Neütralisationskurve von Histidin/Histidin-HCl und N-Acetylhistidin, woraus sich die nutzbaren Pufferkapazitäten im Bereich des physiologischen pH-Wertes; bei N-Acetylhistidin von etwa 60% und bei Histidin von etwa 30% bezogen auf das Verhältnis verbrauchter Base zu Substrat ergeben.

Das genannte Puffersystem kann in der erfindungsgemäßen Lösung in einer Konzentration bevorzugt von etwa 20 mmol/l bis etwa 265 mmol/l enthalten sein.

Fig. 4. Zeigt ein Beispiel für die Toxizität von Histidin unter warmen Bedingungen und Herabsetzung dieser Toxizität durch Flistidinderivate. Kultivierte Rattenhepatozyten wurden 5 h bei 37°C unter aeroben Bedingungen in Gegenwart und Abwesenheit von 198 mM L-Histidin bzw. von 198 mM N□-Acetylhistidin inkubiert. Als Grundlösung diente Krebs-Henseleit-Puffer (KH), eine in Zellkulturen häufig verwendete physiologische Salzlösung; in den Histidin(derivat)-haltigen Lösungen wurde die NaCl-Konzentration um 99 mM vermindert und der pH-Wert durch Zugabe von HCl bzw. NaOH auf pH 7,2 eingestellt.

Diese Weiterentwicklung erlaubt eine noch bessere Erhaltung der zellulären Funktionen nach Konservierung.

Eines der Kationen ist zweckmäßig Natrium. Relativ geringe Natriumkonzentrationen sind zum einen vorteilhaft für die elektramechanische Entkopplung an der Zellmembran (Herzstillstand), zum anderen vermindern sie die hypoxische Schädigung aufgrund der Vermeidung/Verminderung des hypoxieinduzierten Natriurneinstroms. Zudem bleibt eine osmotische Reserve für andere Substanzen (z.B. Puffersubstanzen, s.u.) erhalten. Auch bei niedriger Natriumkonzentration der Lösung ist eine schnelle Einstellung der Homöostase in der Reperfusionsphase möglich, da der interstitielle Anstieg von Natrium (140 mM) schnell erfolgt. Daher empfiehlt sich eine Natriumkonzentration von etwa 10 mmol/l bis etwa 120 mmol/l in der erfindungsgemäßen Lösung.

Eine relativ geringe, jedoch über dem physiologischen extrazellulären Wert liegende Kaliumkonzentration unterstützt die elektromechanische Entkopplung an der Zellmembran (Herzstillstand), vermeidet jedoch die Nachteile stark hyperkalämischer Lösungen (stark hyperkalämische Lösungen induzieren einen schnellen Herzstillstand, sind aber auch in der Reoxygenierungsphase verantwortlich für Rhythmusstörungen und ggf. verstärkte Reperfusionsschäden; in der Reperfusionsphase ist die Einstellung der interstitiellen Kaliumkonzentration zeitlich verzögert, wodurch die Quote verspäteter Initialfunktionen der Organe bei diesen Lösungen erhöht ist). Nur leicht hyperkalämische Lösungen besitzen zudem den Vorteil, dass ein Auswaschen der Lösung aus dem zu transplantierenden Organ vor Reperfusion nicht erforderlich ist (die Lösungen können also ihre protektiven Eigenschaften bis zum Ende der Ischämiezeit entfalten), und verfügen über eine hohe systemische Tolerabilität. Daher empfiehlt sich ein Kaliumkonzentration von etwa 5 mmol/l bis etwa 25 mmol/l in der erfindungsgemäßen Lösung.

Eine deutlich erhöhte Magnesiumkonzentration (über den Serum-Normwert hinaus) wird angestrebt, da Magnesium als Cofaktor zahlreicher glykolytischer Enzymsysteme den anaeroben Stoffwechsel stützt und somit zur Bildung von ATP auch während der kalten Ischämie beiträgt. Zudem trägt Magnesium zur Aufrechterhaltung der Aktivität der Na⁺-K⁺-ATPase der Plasmamembran bei und wirkt so den Veränderungen der Ionenhomöostase entgegen. Als physiologischer Ca²⁺-Antagonist kann Magnesium außerdem den schädlichen Effekten einer intrazellulären Calciumakkumulation entgegenwirken. In der Reperfusionsphase ist ein hoher Pool an Magnesium erforderlich, um den aeroben Energiestoffwechsel zu stimulieren. Die gefäßdilatativen Eigenschaften von Magnesium wirken außerdem den konstriktiven Effekten beim Reperfusionsschaden entgegen. Daher empfiehlt sich eine Magnesiumkonzentration von etwa 3 mmol/l bis etwa 27 mmol/l in der erfindungsgemäßen Lösung.

Unter ischämischen Bedingungen (und der damit verbundenen intrazellulären Azidose und Störung der Natriumhomöostase) besteht das Risiko eines cytosolischen Calciumanstiegs. Dies führt zum Anstieg unerwünschter Aktivitäten, z. B. Stimulation von Proteasen und Phospholipasen, und geht beim Herzen mit einer Hyperkontraktur mit hohem ATP-Verbrauch einher. Die physiologischen Funktionen von Calcium sind somit während der Ischämie weitestgehend zu unterdrücken, weswegen sich die Calciumkonzentration in der Lösung im Bereich der cytoplasmatischen Konzentrationen bewegen sollte. Daher empfiehlt sich eine freie Calciumkonzentration von etwa 0,0001 mmol/l bis etwa 1,5 mmol/l in der erfindungsgemäßen Lösung.

Die basischen Aminosäuren Lysin und Arginin bzw. ihre Derivate als Dipeptide mit Glycin (Lys-Gly, Gly-Lys bzw. Arg-Gly, Gly-Arg) können zur Deckung fehlender Basenäquivalente herangezogen werden, da die Konzentration und das Verhältnis der Kationen Natrium. Kalium, Magnesium und Calcium auf Grund bereits beschriebener Tatsachen festgelegt ist. Die Konzentration an Lysin und/oder Lysinderivaten bzw. Arginin und/oder Argininderivaten in der erfindungsgemäßen Lösung kann jeweils bis zu etwa 140 mmol/l betragen.

Das physiologische extrazelluläre Anion Chlorid wird in einer im Vergleich zu physiologischen Werten deutlich erniedrigten Konzentration eingesetzt, um Störungen der Ionenhomöostase während der kalten Ischämie zu vermindern. Dies gilt insbesondere für einen hypoxieinduzierten Einstrom von Kationen wie dem Natrium. Aus Gründen der Elektroneutralität ist dieser Einstrom vom parallelen Einstrom von Anionen, bevorzugt von Chlorid, abhängig. Hinweise verschiedener Autoren lassen darauf schließen. dass hohe Chlorid-Konzentrationen das Ausmaß eines intrazellulären Ödems begünstigen. Daher empfiehlt sich eine Chlorid-Konzentration in der erfindungsgemäßen Lösung möglichst weit unterhalb des physiologischen Niveaus zu halten, bis maximal 90 mmol/l. Neben dem Chlorid kann auch Lactobionat als impermeables Anion in einer Konzentration bis zu etwa 140 mmol/l zugesetzt werden.

Die in den organprotektiven Lösungen eingesetzten Konzentrationen an Anionen ergeben sich im allgemeinen aus der Wahl der Kationen und des verwendeten (an)organischen Puffers. Die Verwendung von impermeablen Anionen, wie z.B. Lactobionat, soll der Entstehung eines intrazellulären Ödems entgegenwirken. Aspartat stellt eine hervorragende Alternative dar, zumal mit der Verwendung von Aspartat mehrere Ziele verfolgt werden können:
a) Aspartat als Säureäquivalent ersetzt das unvorteilhafte Chlorid.
b) Die Asparaginsäure unterstützt aktiv den Stoffaustausch an Membranen und beschleunigt die Wiederherstellung der Homöostase.
c) Aspartat begünstigt in Verbindung mit α-Ketoglutarat den aeroben Energiestoffwechsel in der kritischen Phase der Reperfusion bzw. Reoxygenierung des Organs und beschleunigt dessen Initialfunktionen.

Besonders in der Reoxygenierungsphase, wenn mit zunehmender Erwärmung des Organs der Energiebedarf erheblich ansteigt, ist es notwendig, die Energiebereitstellung zu forcieren, denn ein ATP-Mangel gerade in dieser Erwärmungsphase würde erheblich die Funktionalität des Organs beeinträchtigen und das Ausmaß eines Reperfusionsschadens erhöhen. Es empfiehlt sich eine Aspartat-Konzentration von bis zu etwa 140 mmol/l in der erfindungsgemäßen Lösung. Zweckmäßig ist in der erfindungsgemäßen Lösung Aspartat im Verhältnis zu Chlorid im Überschuss vorhanden.

Die Aminosäure Glycin wird in höherer millimolarer Konzentration eingesetzt, da Glycin in diesen Konzentrationen den hypoxieinduzierten Natriumeinstrom durch eine Stabilisierung der Plasmamembran (Vermeidung eines diffusionsorientierten Stoffaustauschs) verhindert und zudem die Aktivierung von Makrophagen hemmt. Empfehlenswert ist eine Glycin Konzentration von bis zu etwa 30 mmol/l in der erfindungsgemäßen Lösung.

Auch während der kalten Ischämie ist der Energiebedarf des betreffenden Organs nicht unerheblich, weswegen zur Unterstützung energieliefernde Substrate oder energiestoffwechselfördernde Substanzen der Lösung zuzugeben sind. Verschiedene Konzepte werden dabei verfolgt (s. auch Aspartat, s.o., und α-Ketoglutarat, s.u.), eines davon ist der Zusatz von Glucose.

Eine physiologische Glucosekonzentration der Lösung erlaubt auch den Zellen, die aufgrund vergleichsweise geringer Glycogenvorräte auf exogenes Glucoseangebot angewiesen sind, wie z.B. Endothelzellen, die Energiegewinnung über anaerobe Glycolyse während der Ischämie. Die Glucosekonzentration ist jedoch so gewählt, dass eine exzessive Glucoseaufnahme durch andere Zellen, insbesonders durch Hepatozyten (ein Problem bei früheren Konservierungslösungen mit extrem hohen Glucosekonzentrationen), vermieden wird. Daher können die erfindungsgemäßen Lösungen bis zu 10 mmol/l Glucose enthalten. α-Ketoglutarat dient zusammen mit Aspartat zur Unterstützung des Metabolismus unter/nach hypoxischen Bedingungen. Man wählt zweckmäßig eine Konzentration von etwa 1 mmol/l bis etwa 9 mmol/l.

Der Zusatz von osmotisch wirksamen Substanzen ist in dem Maße erforderlich, wie es zum Erreichen des erforderlichen physiologischen osmotischen Drucks von ca. 300 mosm/L notwendig ist. Im allgemeinen werden Polyole/Zucker (z.B. Mannitol, Raffinose, Saccharose) oder auch hochmolekulare Substanzen (z.B. HES, Dextran) hierzu verwendet. Letztere haben sich für einige Organe nicht bewährt, da die Nachteile der mit HES und Dextran erzeugten hohen Viskosität die Qualität der Protektion beeinträchtigen.
Viskosität bei 4 °C :
Custodiol® 1,8 cP
UW - Lösung 4,8 cP

Als Osmolyte sollen je nach Organ Mannitol, Xylitol, Sorbitol, Saccharose oder Raffinose zum Einsatz kommen. Die Osmolyt-Konzentration beträgt zweckmäßig bis zu 140 mmol/l in der erfindungsgemäßen Lösung.

Zur Vermeidung eines interstitiellen Ödems ist in einigen Organen/Geweben der Zusatz einer kolloidosmotisch wirksamen Substanz vorteilhaft, z.B. Dextran-40/Dextran-70. Dies gilt im besonderen Maße für Lunge- und Pankreasprotektion.

Zum Einfrieren von Zellen und Geweben ist weiterhin der Zusatz von zusätzlichen Cryoprotektiva, wie z.B. Dimethylsulfoxid (DMSO) von Vorteil.

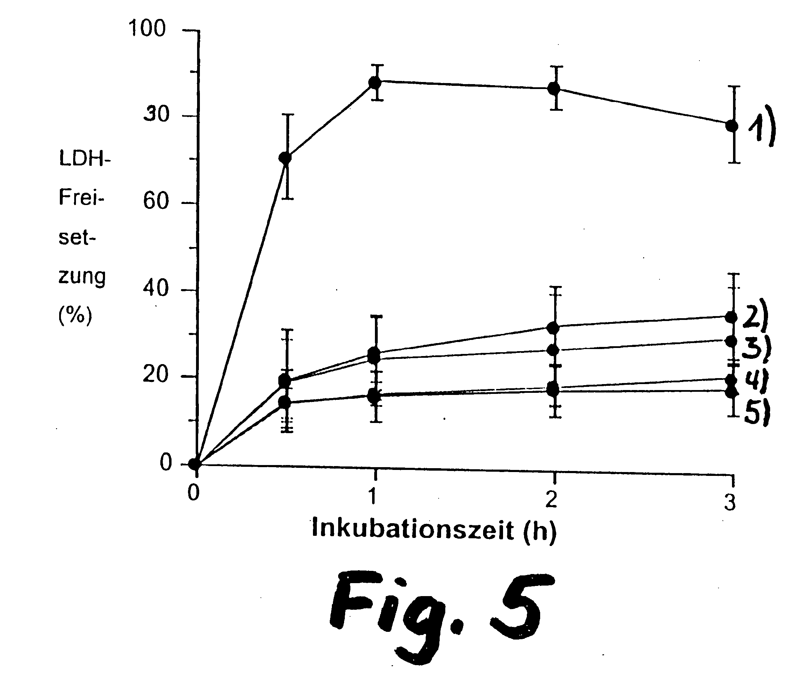

Fig. 5 zeigt die Zellschädigung von Hepatozyten durch Wasserstoffperoxid und deren Schutz mit verschiedenen Hydroxamsäuren, und zwar betreffen:
Kurve 1) N4 + 20 mM/l H₂O₂
Kurve 2) N4 + 20 mM/l H₂O₂ + 1 mM/l 4-Chlor-N-methyl-benzhydroxamsäure
Kurve 3) N4 + 20 mM/l H₂O₂ + 1 mM/l 2-Hydroxy-3-methyl-isocarbosteryril
Kurve 4) N4 + 20 mM/l H₂O₂ + 1 mM/l 3,4,5-Trimethoxy-N-methyl-benzhydroxamsäure
Kurve 5) N4 + 20 mM/l H₂O₂ + 1 mM/l 3,4-Dimethoxy-N-methyl-hydroxamsäure,
wobei N4 die Lösung gemäß Beispiel II, jedoch ohne Trolox und Hydroxamsäurederivat bedeutet.

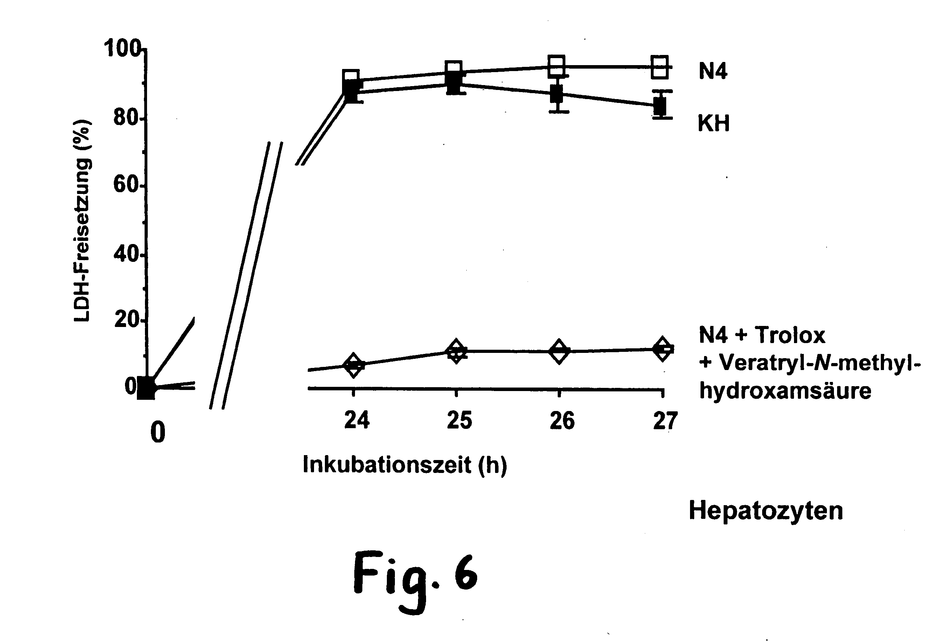

Fig. 6 zeigt die Hemmung der Kälteschädigung des Hepatozyten durch die neue Konservierungslösung. Kultivierte Rattenhepatozyten wurden für 24h bei 4°C in einer erfindungsgemäßen Lösung mit N-Actylhistidin als Puffer und unter Zusatz von Trolox, 1mM/l, und dem Hydroxamsäurederivat Veratryl-N-methylhydroxamsäure, 0,5 mM/l und zum Vergleich in Krebs-Henseleit-Puffer (KH) inkubiert. Die kalte Inkubation erfolgte unter aeroben Bedingungen; nach 24h wurden die Zellen zur Simulation einer Reperfusion in Zellkulturmedium bei 37°C wiedererwärmt. Als Indikator einer Zellschädigung diente die Freisetzung cytosolischer Lactatdehydrogenase (LDH).

### Beispiele

Im Folgenden sind Beispiele von Lösungen dargestellten, die den Anforderungen des hier beanspruchten Schutzumfangs entsprechen.

### Beispiel I:

| | **mmol/l** |
|---|---|
| Na(+) | 25 |
| K(+) | 15 |
| Mg(++) | 10 |
| Ca(++) | 0,1 |
| Cl(-) | 25 |
| Aspartat(-) | 33 |
| Ac-N-His | 60 |
| Ac-N-His (-) | 60 |
| Lysin-H(+) | 60 |
| Glycin | 10 |
| Tryptophan | 2 |
| Veratryl-N-methylhydroxamsäure | 2 |
| Trolox | 2 |
| Ketoglutarat(-) | 2 |
| N-Methylsalicylhydroxamsäure | 2 |
| pH | 7,2 |
| Osmolalität | 310 |

### Beispiel II:

| | mmol /l |
|---|---|
| Na(+) | 15 |
| K(+) | 10 |
| Mg(++) | 8 |
| Ca(++) | 0,015 |
| Cl(-) | 0,03 |
| Aspartat(-) | 38 |
| Ac-N-His | 60 |
| Ac-N-His (-) | 60 |
| Lysin-H(+) | 60 |
| Glycin | 10 |
| Tryptophan | 2 |
| 2,3-Dimethoxy-N-methylbenzhydroxamsäure | 2 |
| Trolox | 2 |
| Ketoglutarat(-) | 3 |
| N-Methylsalicylhydroxamsäure | 2 |
| Mannitol | 30 |
| pH | 7,2 |
| Osmolalität | 310 |

### Beispiel III:

| | **mmol/l** |
|---|---|
| Na(+) | 15 |
| K(+) | 10 |
| Mg(++) | 16 |
| Ca(++) | 0,04 |
| Cl(-) | 0,03 |
| Aspartat(-) | 54 |
| Ac-N-His | 60 |
| Ac-N-His (-) | 60 |
| Lysin-H(+) | 60 |
| Glycin | 6 |
| Tryptophan | 2 |
| 2,3-Dimethoxy-benzhydroxamsäure | 1 |
| Trolox-OCH₃ | 2 |
| Ketoglutarat(-) | 3 |
| 2-Hydroxy-3-methyl-isocarbostryril | 1 |
| Mannitol | 20 |
| pH | 7,2 |
| Osmolalität | 310 |

### Beispiel IV:

| | **mmol/l** |
|---|---|
| Na(+) | 15 |
| K(+) | 10 |
| Mg(++) | 8 |
| Ca(++) | 0,015 |
| Cl(-) | 0,03 |
| Aspartat(-) | 31 |
| Ac-N-His | 70 |
| Ac-N-His (-) | 70 |
| Lysin-H(+) | 70 |
| Glycin | 8 |
| Tryptophan | 2 |
| 2-Hydroxy-3-methyl-isocarbostryril | 2 |
| Ketoglutarat(-) | 2 |
| N-Methylsalicylhydroxamsäure | 2 |
| Mannitol | 20 |
| pH | 7,2 |
| Osmolalität | 310 |

### Beispiel V:

| | **mmol/l** |
|---|---|
| Na(+) | 16 |
| K(+) | 10 |
| Mg(++) | 8 |
| Ca(++) | 0,05 |
| Cl(-) | 0,1 |
| Aspartat | 16 |
| N-Ac-His | 80 |
| Histidin | 88 |
| L-Arginin | 12 |
| Glycin | 20 |
| L-Alanin | 10 |
| Tryptophan | 2 |
| Alpha-Ketoglutarat | 3 |
| Mannitol | 40 |
| 3,4-Dimethoxy-N-methyl-hydroxamsäure | 2 |
| pH | 7,1 |
| Osmolalität | 307 |

Entsprechend den funktionalen Bestandteilen einer organprotektiven Lösung, nämlich Puffersystem, Elektrolyte, protektiv wirkende Substanzen und Osmolyte, wird die erfindungsgemäße Lösung hergestellt. Dazu werden in ca. 90% der benötigten Wassermenge zunächst die Puffersubstanzen gelöst. Nun werden die als Elektrolyten notwendigen Neutralsalze der Kationen, also beispielsweise Natrium, Kalium, Magnesium und Calcium, in den angegebenen, physiologisch sinnvollen Konzentrationen zugegeben und unter Rühren gelöst. Es folgen als weitere Bestandteile die protektiv wirksamen Substanzen, die der Lösung zugegeben werden. Danach wird der pH-Wert der Lösung überprüft und gegebenen falls auf den im Patentanspruch 1 angegebenen Wert eingestellt. Schließlich wird die angegebene Menge Osmolyte zugegeben und die Lösung auf das Sollvolumen verdünnt. Nach dem Abfüllen in geeignete Behältnisse wird die Lösung sterilisiert.

Die Herstellung cyklischer Hydoxamsäuren, beispielsweise die sich vom 2-Hydroxypyridin-N-Oxid ableitenden Verbindungen ist beschrieben beispielsweise in Organic Synthesis Collektiv Volume V Seite 623 und folgende. Ein Herstellungsverfahren für Hydroxamsäuren aus Carbonsäurederivaten findet sich in Houben-Weyl, Methoden der organischen Chemie, Seite 686 und folgende, wobei als Lösungsmittel niedere Alkohole DMF, THF benutzt werden. Die Umsetzung mit dem entsprechenden Carbonsäureester kann basenkatalysiert (NaOMe, K₂CO₃, CaO) erfolgen. Schließlich wird zur Herstellung cyklischer Hydoxamsäuren Bezug genommen auf A. Kleemann, J. Engel. Pharmazeutische Wirkstoffe, zweite Auflage 1982, Seite 206 und folgende.

## Patentansprüche

1. Protektive Lösung zur Vermeidung von Lagerungs- oder Ischämie- oder Reperfusionsschäden an Organen, oder an isolierten Zellsystemen oder Gewebteilen nach Perfusion, Operation, Transplantation oder Kryokonservierung und anschließender Reperfusion, welche als Elektrolyte Alkali-, gegebenenfalls auch Erdalkaliionen, einen Puffer sowie ein Polyol und/oder einen Zucker enthält, eine Osmolarität von 290 mosm/l bis 350 mosm/l sowie einen pH-Wert von 6,8 bis 7,4 aufweist, sowie mit einem Hydroxamsäurederivat, bei dem das Wasserstoffatom am Hydroxamsäurekohlenstoff alkyl- oder aryl- oder alkylaryl-substituiert ist mit C₁ bis C₂₀, wobei dieser Substituent Heteroatome oder Hydroxy- oder Amino- oder Methoxy-Gruppen enthält, und wobei das Wasserstoffatom am Hydroxamsäurestickstoff alkyl- oder aryl- oder alkylary-substituiert ist mit C₁ bis C₂₀.

2. Lösung nach Anspruch 1, bei der die Substituenten am Hydroxamsäurestickstoff mit den Substituenten am Hydroxamsäurekohlenstoff zu einem Ring geschlossen sind.

3. Lösung nach einem der Ansprüche 1 oder 2, die eine oder mehrere der Verbindungen und/oder deren Salze aus der Gruppe
2-Hydroxy-N-methylbenzhydroxamsäure,
2-Hydroxy-N-benzylbenzhydroxamsäure,
3,4-Dimethoxy-N-methylbenzhydroxamsäure,
2,3-Dimethoxy-N-methylbenzhydroxamsäure,
2,4-Dimethoxy-N-methylbenzhydroxamsäure,
3,5-Dimethoxy-N-methylbenzhydroxamsäure,
3,4,5-Trimethoxy-N-methylbenzhydroxamsäure,
2-Hydroxy-3-methyl-isocarbostyril,
4-Chlor-N-methylbenzhydroxamsäure,
6-Cyclohexyl-1-hydroxy-4-methyl-2(1H)-pyridon
enthält.

4. Lösung nach einem oder mehreren der vorstehenden Ansprüche mit einem Zusatz von Deferoxamin.

5. Lösung nach einem oder mehreren der vorstehenden Ansprüche mit einem Zusatz an einem Derivat der 6-Hydroxy-2,5,7,8-tetramethylchroman-2-carbonsäure.

6. Lösung nach Anspruch 5, in welcher das Derivat der entsprechende Methylester ist.

7. Lösung nach einem der vorstehenden Ansprüche mit einem Kationengehalt an Lysin und/oder Lysinderivat.

8. Lösung nach Anspruch 7 mit einem Kationengehalt an lysinhaltigen Dipeptiden.

9. Lösung nach einem der vorstehenden Ansprüche mit einem Anionengehalt an Aspartat.

10. Lösung nach einem der vorstehenden Ansprüche, in welcher die Hydroxamsäure-Derivate in einer Konzentration von bis zu 10_mmol/l enthalten sind.

11. Lösung nach einem der Ansprüche 5 bis 10, in welcher Deferoxamin in einer Konzentration von bis zu 10 mmol/l enthalten ist.

12. Lösung nach einem der Ansprüche 5 bis 10, in welcher Trolox in einer Konzentration von bis zu 10 mmol/l enthalten ist.

13. Lösung nach einem der Ansprüche 5 bis 12, in welcher das N-Acetylhistidin in einer Konzentration von 20 mmol/l bis 265 mmol/l enthalten ist.

14. Lösung nach einem der vorstehenden Ansprüche, in welcher als Eletrolyt Natrium zweckmäßig in einer Konzentration von 10 mmol/l bis 120 mmol/l enthalten ist.

15. Lösung nach einem der vorstehenden Ansprüche, in welcher als Elektrolyt Kalium zweckmäßig in einer Konzentration von 5 mmol/l bis 25 mmol/l enthalten ist.

16. Lösung nach einem der vorstehenden Ansprüche, in welcher Magnesium zweckmäßig in einer Konzentration von 3 mol/l bis 27 mmol/l enthalten ist.

17. Lösung nach einem der vorstehenden Ansprüche, in welcher Calcium zweckmäßig in einer freien Konzentration von 0,0001 mmol/l bis 1,5 mmol/l enthalten ist.

18. Lösung nach einem der vorstehenden Ansprüche, in welcher als Kation Lysin und/oder dessen Derivate,
zweckmäßig in einer Konzentration bis zu 140 mmol/l enthalten ist.

19. Lösung nach einem der vorstehenden Ansprüche, in welcher das Aspartat in einer Konzentration von bis zu 140 mmol/l enthalten ist.

20. Lösung nach einem-der vorstehenden Ansprüche, in welcher Chlorid enthalten ist.

21. Lösung nach Anspruch 19 und 20, in welcher das Aspartat im Verhältnis zum Chlorid im Überschuss enthalten ist.

22. Lösung nach einem der vorstehenden Ansprüche, in welcher α-Ketoglutarat zweckmäßig in einer Konzentration von 1 mmol/l bis 9 mmol/l enthalten ist.

23. Lösung nach einem der vorstehenden Ansprüche, in welcher ein Osmolyt zweckmäßig in einer Konzentration von bis zu 140 mmol/l enthalten ist.

24. Verfahren zur Herstellung einer Lösung nach einem der vorstehenden Ansprüche, bei welchem in einem Überschuss an Wasser, zweckmäßig in 90 % der benötigten Wassermenge, die Elektrolyten unter Rühren aufgelöst, der Puffer und anschließend die Hydroxamsäure-Derivate zugegeben werden, sodann der pH-Wert eingestellt wird, der oder die Osmolyte zugegeben und die Lösung auf das Sollvolumen mit Wasser aufgefüllt wird.

25. Verfahren nach Anspruch 24, bei welchem Hydroxamsäure-Derivate verwendet werden, zu deren Gewinnung niedere Alkohole- und/oder DMF und/oder THF als Lösungsmittel eingesetzt wurden.

26. Verfahren nach Anspruch 25, bei welchem die Umsetzung mit einem Carbonsäureester basenkatalysiert durchgeführt wurde.

## Claims

1. Protective solution for preventing storage or ischemic or reperfusion damage to organs or to isolated cell systems or to tissue components after perfusion, operation, transplantation or cryopreservation and subsequent reperfusion, which contains alkali ions, possibly also alkaline earth ions as electrolytes, a buffer as well as a polyol and/or a sugar, has an osmolarity of 290 mosm/l to 350 mosm/l as well as a pH value of 6.8 to 7.4, and also having a hydroxamic acid derivative, wherein the hydrogen atom on the hydroxamic acid carbon is alkyl- or aryl- or alkylaryl-substituted with C₁ to C₂₀, wherein this substituent contains heteroatoms or hydroxy or amino or methoxy groups, and wherein the hydrogen atom on the hydroxamic acid nitrogen is alkyl- or aryl- or alkylaryl-substituted with C₁ to C₂₀.

2. Solution as claimed in Claim 1, wherein the substituents on the hydroxamic acid nitrogen are cyclised with the substituents on the hydroxamic acid carbon.

3. Solution as claimed in any one of Claims 1 or 2, containing one or more of the compounds and/or salts thereof from the group consisting of
2-hydroxy-N-methyl-benzohydroxamic acid,
2-hydroxy-N-benzyl-benzohydroxamic acid,
3,4-dimethoxy-N-methyl-benzohydroxamic acid,
2,3-dimethoxy-N-methyl-benzohydroxamic acid,
2,4-dimethoxy-N-methyl-benzohydroxamic acid,
3,5-dimethoxy-N-methyl-benzohydroxamic acid,
3,4,5-trimethoxy-N-methyl-benzohydroxamic acid,
2-hydroxy-3-methyl-isocarbostyril,
4-chloro-N-methyl-benzohydroxamic acid,
6-cyclohexyl-1-hydroxy-4-methyl-2(1H)-pyridone.

4. Solution as claimed in any one or several of the preceding Claims, having an addition of deferoxamine.

5. Solution as claimed in any one or several of the preceding Claims, having an addition of a derivative of 6-hydroxy-2,5,7,8-tetramethylchroman-2-carboxylic acid.

6. Solution as claimed in Claim 5, wherein the derivative is the corresponding methyl ester.

7. Solution as claimed in any one of the preceding Claims, having a cation content of lysine and/or lysine derivative.

8. Solution as claimed in Claim 7, having a cation content of lysine-containing dipeptides.

9. Solution as claimed in any one of the preceding Claims, having an anion content of aspartate.

10. Solution as claimed in any one of the preceding Claims, wherein the hydroxamic acid derivatives are contained in a concentration of up to 10 mmol/l.

11. Solution as claimed in any one of Claims 5 to 10, wherein deferoxamine is contained in a concentration of up to 10 mmol/l.

12. Solution as claimed in any one of Claims 5 to 10, wherein trolox is contained in a concentration of up to 10 mmol/l.

13. Solution as claimed in any one of Claims 5 to 12, wherein the N-acetylhistidine is contained in a concentration of 20 mmol/l to 265 mmol/l.

14. Solution as claimed in any one of the preceding Claims, wherein as the electrolyte sodium is expediently contained in a concentration of 10 mmol/l to 120 mmol/l.

15. Solution as claimed in any one of the preceding Claims, wherein as the electrolyte potassium is expediently contained in a concentration of 5 mmol/l to 25 mmol/l.

16. Solution as claimed in any one of the preceding Claims, wherein magnesium is expediently contained in a concentration of 3 mmol/l to 27 mmol/l.

17. Solution as claimed in any one of the preceding Claims, wherein calcium is expediently contained in a free concentration of 0.0001 mmol/l to 1.5 mmol/l.

18. Solution as claimed in any one of the preceding Claims, wherein as the cation lysine and/or its derivatives is/are expediently contained in a concentration of up to 140 mmol/l.

19. Solution as claimed in any one of the preceding Claims, wherein the aspartate is contained in a concentration of up to 140 mmol/l.

20. Solution as claimed in any one of the preceding Claims, wherein chloride is contained.

21. Solution as claimed in Claim 19 and 20, wherein the aspartate is contained at a ratio in excess of the chloride.

22. Solution as claimed in any one of the preceding Claims, wherein α-ketoglutarate is expediently contained in a concentration of 1 mmol/l to 9 mmol/l.

23. Solution as claimed in any one of the preceding Claims, wherein an osmolyte is expediently contained in a concentration of up to 140 mmol/l.

24. Method for producing a solution in accordance with any one of the preceding Claims, wherein the electrolytes are dissolved under agitation in an excess of water, expediently in 90% of the required amount of water, the buffer and subsequently the hydroxamic acid derivatives are added, then the pH value is adjusted, the osmolyte(s) is/are added and the solution is made up to the desired volume using water.

25. Method as claimed in Claim 24, wherein hydroxamic acid derivatives which had been obtained using lower alcohols and/or DMF and/or THF as the solvent are used.

26. Method as claimed in Claim 25, wherein the reaction was performed in a base-catalysed manner with a carboxylic acid ester.

## Revendications

1. Solution protectrice destinée à protéger contre des dommages de stockage, ischémiques ou de reperfusion des organes ou des systèmes cellulaires isolés ou des parties de tissus après une perfusion, une opération, une transplantation ou une cryoconservation et une reperfusion consécutive, qui contient en tant qu'électrolytes des ions alcalins et éventuellement aussi des ions alcalino-terreux, un tampon ainsi qu'un polyol et/ou un sucre, qui présente une osmolarité allant de 290 mosm/l à 350 mosm/l ainsi qu'une valeur du pH allant de 6,8 à 7,4, et qui contient un dérivé d'acide hydroxamique dans lequel l'atome d'hydrogène sur le carbone de l'acide hydroxamique est substitué par un alkyle ou un aryle ou un alkylaryle en C₁ à C₂₀, dans laquelle ce substituant contient des hétéroatomes ou des groupes hydroxy, amino ou méthoxy, et dans laquelle l'atome d'hydrogène sur l'azote de l'acide hydroxamique est substitué par un alkyle, un aryle ou un alkylaryle en C₁ à C₂₀.

2. Solution selon la revendication 1, dans laquelle les substituants sur l'azote de l'acide hydroxamique forment un cycle avec les substituants sur le carbone de l'acide hydroxamique.

3. Solution selon l'une des revendications 1 et 2, qui contient un ou plusieurs des composés et/ou de leurs sels choisis dans le groupe comprenant :
l'acide 2-hydroxy-N-méthylbenzhydroxamique,
l'acide 2-hydroxy-N-benzylbenzhydroxamique,
l'acide 3,4-diméthoxy-N-méthylbenzhydroxamique,
l'acide 2,3-diméthoxy-N-méthylbenzhydroxamique,
l'acide 2,4-diméthoxy-N-méthylbenzhydroxamique,
l'acide 3,5-diméthoxy-N-méthylbenzhydroxamique,
l'acide 3,4,5-triméthoxy-N-méthylbenzhydroxamique,
le 2-hydroxy-3-méthyl-isocarbostyrile,
l'acide 4-chloro-N-méthylbenzhydroxamique,
la 6-cyclohexyl-1-hydroxy-4-méthyl-2(1H)-pyridone.

4. Solution selon une ou plusieurs des revendications précédentes, contenant un ajout de déféroxamine.

5. Solution selon une ou plusieurs des revendications précédentes, contenant un ajout d'un dérivé de l'acide 6-hydroxy-2,5,7,8-tétraméthylchroman-2-carboxylique.

6. Solution selon la revendication 5, dans laquelle le dérivé est l'ester méthylique correspondant.

7. Solution selon l'une des revendications précédentes, contenant une teneur en cations de la lysine et/ou d'un dérivé de la lysine.

8. Solution selon la revendication 7, contenant une teneur en cations de dipeptides contenant de la lysine.

9. Solution selon l'une des revendications précédentes, contenant une teneur en anions de l'aspartate.

10. Solution selon l'une des revendications précédentes, dans laquelle les dérivés d'acide hydroxamique sont contenus dans une concentration allant jusqu'à 10 mmol/l.

11. Solution selon l'une des revendications 5 à 10, dans laquelle la déféroxamine est contenue dans une concentration allant jusqu'à 10 mmol/l.

12. Solution selon l'une des revendications 5 à 10, dans laquelle le Trolox est contenu dans une concentration allant jusqu'à 10 mmol/l.

13. Solution selon l'une des revendications 5 à 12, dans laquelle la N-acétylhistidine est contenue dans une concentration allant de 20 mmol/l à 265 mmol/l.

14. Solution selon l'une des revendications précédentes, dans laquelle du sodium, en tant qu'électrolyte, est contenu avantageusement dans une concentration allant de 10 mmol/l à 120 mmol/l.

15. Solution selon l'une des revendications précédentes, dans laquelle du potassium, en tant qu'électrolyte, est contenu avantageusement dans une concentration allant de 5 mmol/l à 25 mmol/l.

16. Solution selon l'une des revendications précédentes, dans laquelle du magnésium est contenu avantageusement dans une concentration allant de 3 mmol/l à 27 mmol/l.

17. Solution selon l'une des revendications précédentes, dans laquelle du calcium est contenu avantageusement dans une concentration libre allant de 0,0001 mmol/l à 1,5 mmol/l.

18. Solution selon l'une des revendications précédentes, dans laquelle de la lysine et/ou des dérivés de celle-ci, en tant que cation, est contenue avantageusement dans une concentration allant jusqu'à 140 mmol/l.

19. Solution selon l'une des revendications précédentes, dans laquelle l'aspartate est contenu dans une concentration allant jusqu'à 140 mmol/l.

20. Solution selon l'une des revendications précédentes, qui contient du chlorure.

21. Solution selon les revendications 19 et 20, dans laquelle l'aspartate est contenu en excès par rapport au chlorure.

22. Solution selon l'une des revendications précédentes, dans laquelle de l'α-cétoglutarate est contenu avantageusement dans une concentration allant de 1 mmol/l à 9 mmol/l.

23. Solution selon l'une des revendications précédentes, dans laquelle un osmolyte est contenu avantageusement dans une concentration allant jusqu'à 140 mmol/l.

24. Procédé pour la préparation d'une solution selon l'une des revendications précédentes, dans lequel les électrolytes sont dissous sous agitation dans un excès d'eau, avantageusement dans 90 % de la quantité d'eau nécessaire, le tampon et ensuite les dérivés d'acide hydroxamique sont ajoutés, puis la valeur du pH est ajustée, le ou les osmolytes sont ajoutés et la solution est complétée jusqu'au volume de consigne avec de l'eau.

25. Procédé selon la revendication 24, dans lequel sont utilisés des dérivés d'acide hydroxamique, pour l'obtention desquels des alcools inférieurs, et/ou du DMF et/ou du THF ont été utilisés en tant que solvants.

26. Procédé selon la revendication 25, dans lequel la réaction a été conduite sous catalyse basique à l'aide d'un ester d'acide carboxylique.
